**Europäisches Patentamt**

**European Patent Office**

**Office européen des brevets**

(19)

(11) Veröffentlichungsnummer: **0 041 664**
**B1**

(12) **EUROPÄISCHE PATENTSCHRIFT**

(45) Veröffentlichungstag der Patentschrift:
04.12.85

(51) Int. Cl.⁴: **G 10 K 11/34**

(21) Anmeldenummer: **81104130.0**

(22) Anmeldetag: **29.05.81**

(54) **Verfahren zur Herstellung einer Ultraschallwandleranordnung.**

(30) Priorität: **06.06.80 DE 3021449**

(43) Veröffentlichungstag der Anmeldung:
**16.12.81 Patentblatt 81/50**

(45) Bekanntmachung des Hinweises auf die Patenterteilung:
**04.12.85 Patentblatt 85/49**

(84) Benannte Vertragsstaaten:
**AT CH DE FR GB LI NL**

(56) Entgegenhaltungen:
**EP - A - 0 025 092**
**DE - A - 2 530 569**
**DE - B - 2 829 570**
**FR - A - 2 292 978**
**FR - A - 2 349 835**
**US - A - 4 122 725**

(73) Patentinhaber: **Siemens Aktiengesellschaft, Berlin und München Wittelsbacherplatz 2, D-8000 München 2 (DE)**

(72) Erfinder: **Diepers, Heinrich, Dr., Veit-Stoss-Strasse 44, D-8552 Höchstadt (DE)**

## Beschreibung

Die Erfindung bezieht sich auf ein Verfahren zum Herstellen einer Ultraschallwandleranordnung mit einer Matrix von Ultraschallschwingern, die jeweils aus einer Submatrix von säulenförmigen, elektrisch gemeinsam gesteuerten Wandlerelementen bestehen, deren Höhe wesentlich größer ist als ihre Dicke. Sowohl die Zwischenräume zwischen den Ultraschallschwingern als auch die Zwischenräume zwischen den Wandlerelementen sind mit einem Füllstoff gefüllt, dessen akustische Impedanz wesentlich verschieden ist von der akustischen Impedanz des Schwingermaterials der Wandlerelemente. Der Füllstoff bildet eine formschlüssige und mechanisch feste Verbindung zwischen den Wandlerelementen.

Zum Herstellen von Ultraschall-Arrays, beispielsweise für die Anwendung in medizinischen Diagnosegeräten, wird im allgemeinen zunächst ein größeres Schwingerplättchen hergestellt und dann eine elektrische oder zusätzlich noch eine mechanische Aufteilung in beispielsweise linear angeordneten Gruppen von Einzelschwingern vorgenommen. Die elektrische Teilung erfolgt durch Aufbringung von getrennten elektrischen Kontaktflächen, vorzugsweise einer Oberflächenmetallisierung. Anschließend wird über diese elektrische Kontaktfelder die Polarisierung des Schwingermaterials vorgenommen.

Zur Stromversorgung sind die Kontaktfelder mit elektrischen Anschlußleitungen versehen. Die Wandlerelemente werden zwischen einem Dämpfungskörper und einem Anpassungskörper angeordnet.

Ein bekannter Wandlerkamm für ein Ultraschall-Array oder einen sogenannten Compound-Scanner besteht aus einer linearen Anordnung von Ultraschallschwingern. Durch sogenannte Feinunterteilung können diese Ultraschallschwinger in akustisch und mechanisch getrennte Wandlerelemente aufgeteilt werden, die gruppenweise elektrisch parallelgeschaltet und gemeinsam gesteuert werden. Die Breite dieser Wandlerelemente wird vorzugsweise wesentlich geringer als die halbe Wellenlänge ($\lambda/2$) der abgestrahlten bzw. empfangenen Ultraschallwellen gewählt. Wird diese Stäbchenausführung der Wandlerelemente mit zusätzlichen Querspalten versehen, so entsteht eine Matrix von Wandlerelementen (DE-C 2 829 570).

Bei der mechanischen Feinteilung der Ultraschallschwinger ist der Abstand der entstehenden Wandlerelemente gegeben durch die Schnittbreite, die nicht beliebig klein gemacht werden kann. Mit abnehmender Breite der Wandlerelemente steigen somit die Teilungsverluste, weil die Größe der Zwischenräume im Verhältnis zur Breite der Wandlerelemente entsprechend zunimmt. Diese Verluste nehmen noch zu, wenn durch zusätzliche Querteilung eine Matrix von Wandlerelementen hergestellt wird. Außerdem bilden die Wandlerelemente durch ihre mechanische Trennung keine formschlüssige Baueinheit und in einer Ausführungsform der Anordnung mit einer Vielzahl von Wandlerelementen ist die Kontaktierung nur mit einem erheblichen Aufwand möglich.

Der Erfindung liegt die Aufgabe zugrunde, ein Verfahren zum Herstellen einer Ultraschallwandleranordnung mit einer Matrix von Ultraschallschwingern anzugeben, die mit einer großen Anzahl von Wandlerelementen mit sehr geringer Breite und sehr geringem Abstand eine mechanisch feste Baueinheit bildet und die in einfacher Weise derart kontaktiert und gesteuert weden kann, daß sie beispielsweise sowohl einen elektronisch erzeugten Fokus als auch eine veränderbare Apertur ermöglicht.

Die beispielsweise in der medizinischen Diagnostik eingesetzten Ultraschall-Arrays für Abbildungssysteme zum Herstellen von Schnittbildern, sogenannten B-Bildern, sind mit einem durch die Wandlerform gebildeten Fokus in der Längsrichtung des Ultraschall-Arrays versehen und erzeugen elektronisch einen Fokus in der dazu senkrechten Richtung. Der elektronisch erzeugte Fokus kann durch unterschiedlich gesteuerte Erregung der Schwinger in einer Gruppe in der Tiefe, d. h. parallel zur Abstrahlrichtung, verschoben werden. Dies ist jedoch nicht möglich bei einem durch die Wandlerform gebildeten Fokus, der beispielsweise durch eine Zylinderkrümmung der Abstrahlfläche gebildet wird. Diese fehlende Synchronisierbarkeit durch eine zusätzlich elektronisch erzeugte Fokussierung auch in der zweiten Richtung, also anstelle der durch die Wandlerform gebildeten Fokussierung, zu erreichen, erfordert einen hohen technologischen Aufwand.

Die Erfindung löst die erwähnte Aufgabe in einfacher Weise und sie besteht in den Gestaltungsmerkmalen nach dem Kennzeichen des Anspruchs 1. Der Füllstoff stellt eine formschlüssige und mechanisch feste Verbindung zwischen den einzelnen Wandlerelementen her und hat gegenüber dem Schwingermaterial der Wandlerelemente entweder eine wesentlich abweichende Schallgeschwindigkeit oder eine abweichende Dichte. Durch die mechanisch feste Baueinheit der Matrix mit einer Vielzahl von Ultraschallschwingern, die jeweils aus einer Matrix von Wandlerelementen bestehen, erhält man eine hohe Packungsdichte mit einer entsprechend großen Abstrahlfläche. Die Abstände zwischen den Wandlerelementen können nur wenige $\mu$m, beispielsweise 5 $\mu$m, betragen und man erhält somit eine entsprechend hohe Empfindlichkeit.

Die Matrix besteht vorzugsweise aus säulenförmigen Wandlerelementen, deren Höhe senkrecht zu den Flachseiten der Matrix mindestens gleich dem doppelten Betrag der Dicke der Wandlerelemente in Richtung der Flachseiten der Matrix beträgt. Jeweils eine Gruppe von Wandlerelementen, die einen Ultraschallschwinger bilden sollen, kann an ihren Stirnflächen, die an einer der Flachseiten der Matrix liegen, mit einer Metallisierung versehen werden, die als Stromzuführung dient. Durch eine entsprechende elektrische Verbindung werden an den Flachseiten der Matrix die Zeilen und Spalten gebildet und mit einem elektrischen Steuerleiter verbunden.

Zur weiteren Erläuterung der Erfindung wird auf die Zeichnung Bezug genommen. Das Verfahren zum Herstellen der Ultraschallwandleranordnung wird anhand der Fig. 1 bis 3 erläutert. In Fig. 4 ist eine Draufsicht auf eine mit dem Verfahren nach der Erfindung hergestellte Ultraschallwandleranordnung mit der elektrischen Steuerung der Wandlerelemente schematisch veranschaulicht. In Fig. 5 ist eine Anordnung mit durch die Wandlerform gebildeter Fokussierung veranschaulicht.

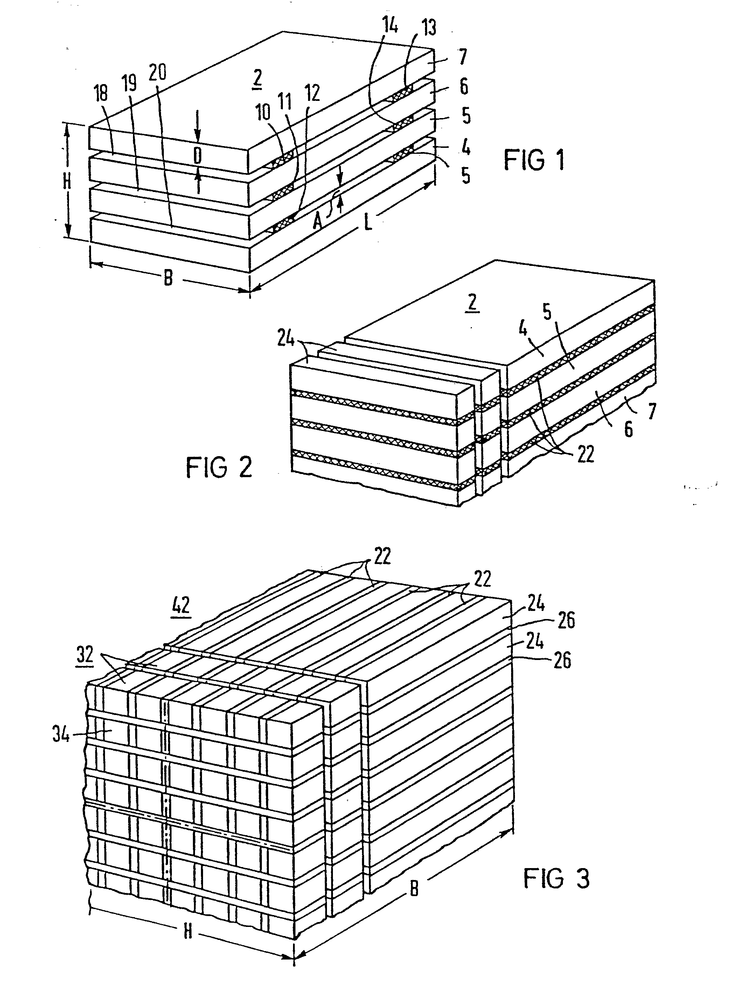

Zum einfachen Herstellen einer Matrix mit den Wandlerelementen wird nach Fig. 1 zunächst eine größere Anzahl von streifenförmigen Platten 4 bis 7 aus Ultraschallschwingermaterial beispielsweise durch Abtrennen aus einem Piezo-Keramikblock hergestellt und zu einem Stapel 2 aufgeschichtet. Die Platten 4 bis 7 können beispielsweise aus Bleizirkonat-Titanat $Pb(ZrTi)O_3$ oder Bleimetaniobat $Pb(NbO_3)_2$ oder einem anderen Schwingermaterial bestehen. Die Dicke der Platten 4 bis 7 kann beispielsweise 100 µm oder weniger betragen. Zwischen den Platten 4 bis 7 können Abstandshalter 10 bis 15 angeordnet werden, die aus Streifen einer Kunststoffolie bestehen können, deren Dicke den Abstand A der Platten bestimmt und wesentlich geringer ist als die Dicke D der Platten 4 bis 7. Die Anzahl der Platten, von denen in der Figur zur Vereinfachung nur vier dargestellt sind, wird gleich der gewünschten Anzahl der Wandlerelemente gewählt, die in der herzustellenden Matrix in einer Reihe angeordnet sein sollen, und kann beispielsweise mehrere 100 betragen. Die Platten 4 bis 7 des Stapels 2 werden von außen fixiert, beispielsweise mittels Klammern, und die Spalten 18 bis 20 zwischen den Platten 4 bis 7 werden mit einem Füllmaterial vesehen, das gegenüber dem Ultraschallschwingermaterial der Platten 4 bis 7 wesentlich abweichende, d. h. eine wesentlich geringere oder auch wesentlich größere akustische Impedanz aufweist, die sich als Produkt aus der Dichte des Ultraschallschwingermaterials und der Schallgeschwindigkeit des in Frage kommenden Schwingungsmods ergibt. Das Füllmaterial kann beispielsweise aus einem selbsthärtenden Kunststoff, vorzugsweise Araldit, oder auch Epoxidharz oder Silikon-Gummi bestehen. Die Spalten 18 bis 20 können mit dem Füllstoff ausgegossen oder auch durch Tauchen des Stapels 2 gefüllt werden.

Nach dem Aushärten des Füllstoffes 22 zwischen den Platten 4 bis 7 entsteht nach Fig. 2 jeweils ein Formkörper 24 durch Abtrennen von Scheiben senkrecht zur Stapelebene und parallel zur Richtung der Breite B und in Richtung der Länge L des Stapels 2 hintereinander. Die Dicke der Formkörper 24 bestimmt die Dicke der Wandlerelemente in der herzustellenden Matrix.

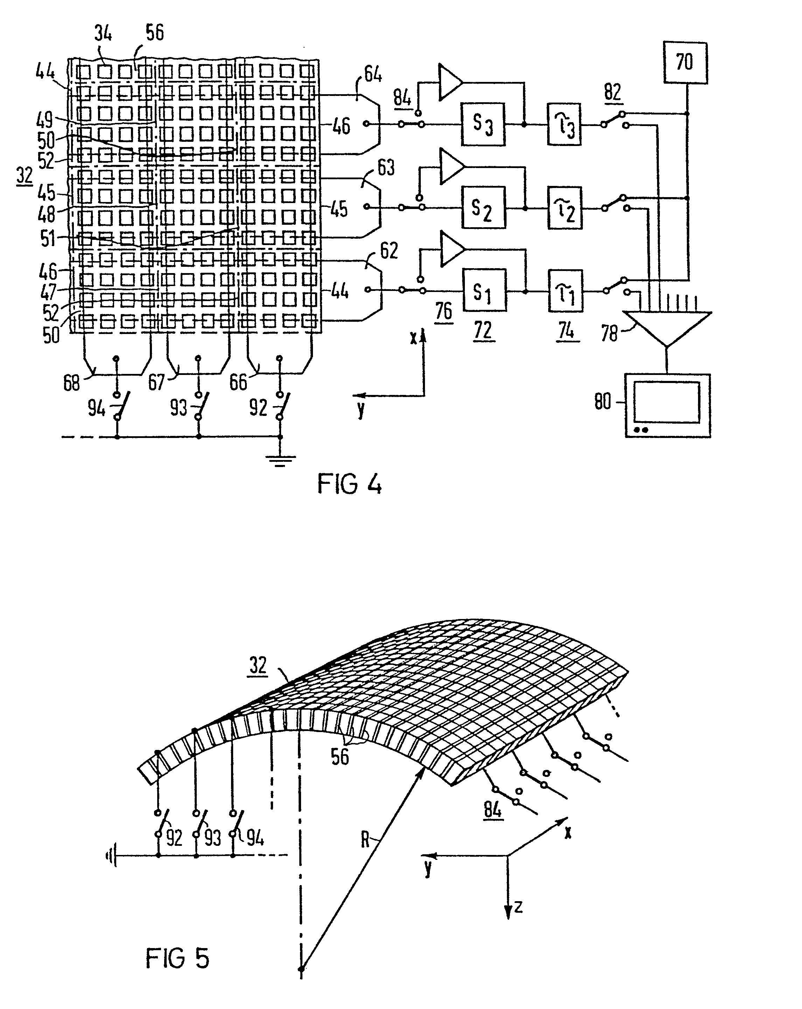

Von diesen Formkörpern 24, die in ihrer Längsrichtung hintereinander jeweils abwechselnd einen Streifen aus Piezomaterial und eine Zwischenschicht des Füllstoffes 22 enthalten, werden nun eine größere Anzahl in der Art gestapelt, wie in Fig. 3 dargestellt ist. Dabei können zunächst Abstandshalter zwischen den Formkörpern 24 angeordnet werden und die dadurch entstehenden Spalten mit einem Füllstoff 26 gefüllt werden, der vorzugsweise aus einem selbsthärtenden Kunststoff bestehen kann.

Nach einem abweichenden Verfahrensmerkmal können die Formkörper 24 auch zunächst auf wenigstens einer ihrer Flachseiten mit einer dünnen Oberflächenschicht des Füllstoffes 26 versehen werden. Der Füllstoff 26 kann beispielsweise aufgespritzt, aufgesprüht oder auch aufgedruckt werden. In diesem Falle kann der Füllstoff 26 selbst eine gut haftende Verbindung der Platten 24 untereinander herstellen oder die mit dem Füllstoff 26 beschichteten Flachseiten der Formkörper 24 können auch miteinander verklebt werden. Nach dem Aushärten des Füllstoffes 26 entsteht jeweils eine Matrix 32 aus Wandlerelementen 34 durch einen Schnitt parallel zur Längsrichtung H und über die Breite B des Stapels 42 hintereinander. Innerhalb der Matrix 32 werden jeweils benachbarte Wandlerelemente 34 an ihren Stirnseiten derart mit einer gemeinsamen elektrischen Kontaktierung versehen, daß sie einen Ultraschallschwinger bilden, wie es in Fig. 3 durch eine strichpunktierte Trennungslinie angedeutet ist. Der Abstand der Schnitte innerhalb des Stapels 42 bestimmt jeweils die Länge der säulenförmigen Wandlerelemente 34 und wird vorzugsweise wenigstens so groß wie die Dicke D der Platten 4 bis 7 gewählt, welche zugleich die Breite der Wandlerelemente 34 bestimmt.

Die elektrische Polarisation des Schwingermaterials kann vor dem Abtrennen der Platten 4 bis 7 von einem Block des gewünschten Schwingermaterials erfolgen. Dann werden in den folgenden Verfahrensschritten die Schnittebenen jeweils so gewählt, daß im Endzustand der hergestellten Matrix 32 nach Fig. 3 die einzelnen Wandlerelemente 34 senkrecht zu den Flachseiten der Matrix 32 polarisiert sind. Die elektrische Polarisation kann jedoch auch vorgenommen werden, wenn die Matrix 32 in Fig. 3 fertiggestellt und die Wandlerelemente 34 elektrisch kontaktiert sind.

Nach Fig. 4 wird eine mit dem Verfahren nach der Erfindung hergestellte Matrix 32 durch Ultraschallschwinger gebildet, von denen in der Figur lediglich einige dargestellt und jeweils mit einer strichpunktierten Umrandung versehen und mit 44 bis 52 bezeichnet sind. Die Ultraschallschwinger bestehen jeweils aus einer Matrix der säulenförmigen Wandlerelemente 34, die an ihren jeweils einer Flachseite der Matrix 32 liegenden Stirnflächen mit einer in der Figur nicht dargestellten gemeinsamen elektrischen Kontaktierung versehen sind und dadurch gemeinsam elektrisch gesteuert werden können. Die Zwischenräume 56 zwischen den Wandlerelementen 34 sind in der Figur zur Verdeutlichung vergrößert dargestellt. An der unteren Flachseite der Matrix 32 sind die Ultraschallschwinger 44, 47 und 50 und 45, 48 und 51 sowie 46, 49 und 52 durch gemeinsame elektrische Verbindung in Zeilen 62 bis 64 angeordnet, was in der Figur durch eine gestrichelte Einfassung der gemeinsamen elektrischen Kontaktierung angedeutet ist. An der oberen Flachseite der Matrix 32 bilden jeweils die Ultraschallschwinger 44 bis 46 sowie 47 bis 49 und 50 bis 52 durch gemeinsame elektrische Kontaktierung Spalten, die mit 66 bis 68 bezeichnet sind. Den Zeilen 62 bis 64 ist eine Anordnung zum Senden von Ultraschallimpulsen zugeordnet, die einen gemeinsamen Taktgeber 70 und für die einzelnen Zeilen 62 bis 64 jeweils einen Sendeverstärker einer Senderkette 72 enthält. Die in der Figur mit $S_1$, $S_2$ und $S_3$ bezeichneten Sendeverstärker erhalten ihre elektrischen Taktimpulse jeweils über einen Umschalter einer elektronischen Weiche 82 und eine elektronische Verzögerungsstufe einer Verzögerungskette 74. Die Verzögerungsstufen sind in der Figur mit $\tau_1$, $\tau_2$ und $\tau_3$ bezeichnet. Die Verzögerungskette 74 ist sowohl der Anordnung zum Senden von Ultraschallimpulsen als auch einem Empfänger zugeordnet, der eine elektronische Verstärkerkette 76 sowie einen Summierverstärker 78 und einen Bildschirm 80 enthält. Die Sendeimpulse werden den Ultraschallschwingern 44 bis 52 jeweils über einen Umschalter einer elektronischen Weiche 84 zugeführt. Die Echoimpulse werden über die Weiche 84 und jeweils eine Verstärkerstufe der Verstärkerkette 76 sowie die betreffende Verzögerungsstufe 74 und den Umschalter der elektronischen Weiche 82 dem Summierverstärker 78 zugeführt und können auf dem Bildschirm 80 sichtbar gemacht werden. Die elektronischen Weichen 82 und 84 können vorzugsweise als integrierte Schaltkreise ausgebildet sein, deren Sperrdämpfung vorzugsweise wenigstens etwa 40 dB betragen kann. Dies gilt insbesondere für die elektronische Weiche 84.

Den Spalten 66 bis 68 sind elektronische Schalter zugeordnet, die in der Figur mit 92 bis 94 bezeichnet sind. Die gemeinsame Kontaktierung jeweils einer der Zeilen 62 bis 64 und Spalten 66 bis 68 besteht jeweils aus einer Metallisierung in Streifenform, die beispielsweise durch Aufsputtern einer Metallschicht aus Chrom-Platin-Gold bestehen kann. Die Breite dieser Streifen ist so bemessen, daß sie alle Wandlerelemente der gemeinsam gesteuerten Ultraschallschwinger bedeckt, die gleichphasig schwingen sollen. Diese Breite beträgt beispielsweise bei einem Ultraschall-Array für eine Frequenz von 2,5 MHz etwa 3 mm. Mehrere Reihen von Ultraschallschwingern elektrisch zusammengeschaltet zu einer Gruppe bilden die Strahlapertur. Durch Fortschaltung dieser Gruppe entlang dem Ultraschall-Array in X-Richtung entsteht Zeile für Zeile das Bild. Die Spalten 66 bis 68 an der oberen Flachseite der Matrix 32 sind dabei über die Schalter 92 bis 94 an gemeinsames Potential gelegt, das im allgemeinen Nullpotential sein wird.

Ein durch die Wandlerform gebildeter Fokus in der Y-Z-Ebene kann nach Fig. 5 beispielsweise durch Biegen der Matrix 32 um ihre Längsachse erzeugt werden. Die erforderliche Krümmung erhält man beispielsweise mit Hilfe von Formkörpern, bevor der Füllstoff 56 zwischen den Wandlerelementen 34 völlig ausgehärtet ist. Unter Umständen kann es zweckmäßig sein, die Krümmung der Matrix 32 bei erhöhter Temperatur von beispielsweise 40°C vorzunehmen. Anschließend erfolgt die vollständige Aushärtung in den Formkörpern unter den von den Herstellern des Füllstoffes 56 angegebenen Bedingungen. Die elektronisch erzeugte Fokussierung in der X-Z-Ebene erhält man durch entsprechende verzögerte Ansteuerung der Zeilen, die über die Umschalter der elektronischen Weiche 84 angeschlossen sind. Die Spalten an der Oberseite der Matrix 32 werden über die Schalter 92 bis 94 an Nullpotential angeschlossen. Der Radius R der Krümmung der Matrix 32 bestimmt die Tiefe des Fokuspunktes innerhalb des in der Figur nicht dargestellten, zu untersuchenden Körpers.

Anstelle der in Fig. 5 angenommenen zylinderförmigen Krümmung mit dem Radius R kann beispielsweise auch eine parabolische oder hyperbolische Krümmung sowie eine Dachform (Axicon-Array) gewählt werden. Durch diese unterschiedlichen Krümmungen erhält man jeweils eine unterschiedlich breite und lange Fokuszone. Sie kann zur weiteren Optimierung der Lage des durch die wählbare Apertur gegebenen Fokus dienen, insbesondere wenn in einem vorbestimmten Tiefenbereich entlang der Abstrahlrichtung eine stärkere Einengung des Schallbündels in der Y-Z-Ebene über den natürlichen Fokus hinaus gewünscht wird. Auch in der ebenen Anordnung der Matrix nach Fig. 4 erhält man eine elektronisch erzeugte Fokussierung in der X-Z-Ebene durch Laufzeitverzögerung der Sende- und Echoimpulse durch die Verzögerungsglieder $\tau_1$ bis $\tau_3$ der Verzögerungskette 74. Einen dynamischen Fokus erhält man durch Änderung der Verzögerung entsprechend der Laufgeschwindigkeit des Ultraschallimpulses.

Die Verzögerung wird so gewählt, daß die Fokuslage jeweils in eine Tiefe gelegt werden kann, aus der man die Pulsantwort erwartet. Die Tiefenlage des natürlichen Fokus in der Y-Z-Ebene ist abhängig von der Größe der Apertur und der Wellenlänge im zu prüfenden Körper. Durch Änderung der Apertur, d. h. durch Steuerung der Spalten 66 bis 68, kann der natürliche Fokus in die Nähe des elektronischen Fokus der Zeilen 62 bis 64 gelegt und mit diesem verändert werden. Zu diesem Zweck werden jeweils in der Y-Richtung am Anfang und am Ende der Matrix 32 liegende Spalten durch Öffnen der Schalter 92 bis 94 elektrisch von der Matrix 32 abgetrennt. Damit erhält man wenigstens annähernd einen Punktfokus. Unter Umständen kann es zweckmäßig sein, mehrere benachbarte Spalten am Anfang und Ende der Matrix in der Y-Richtung gleichzeitig zu- oder abzuschalten.

## Patentansprüche

1. Verfahren zum Herstellen einer Ultraschallwandleranordnung mit einer Matrix von Ultraschallschwingern (4 bis 12), die jeweils aus einer Submatrix von säulenförmigen, elektrisch gemeinsam gesteuerten Wandlerelementen (14) bestehen, deren Höhe wesentlich größer ist als ihre Dicke, und bei der sowohl die Zwischenräume zwischen den Ultraschallschwingern (4 bis 12) als auch die Zwischenräume zwischen den Wandlerelementen (14) mit einem Füllstoff (16) gefüllt sind, dessen akustische Impedanz wesentlich verschieden ist von der akustischen Impedanz des Schwingermaterials der Wandlerelemente (14) und der eine formschlüssige und mechanisch feste Verbindung zwischen den Wandlerelementen (14) bildet, dadurch gekennzeichnet, daß Platten (64 bis 67) aus Ultraschallschwingermaterial mit ihren Flachseiten jeweils abwechselnd mit einer dünnen Schicht aus einem Füllstoff (82), dessen akustische Impedanz wesentlich verschieden ist von der akustischen Impedanz des Ultraschallschwingermaterials der Platten (64 bis 67), aufeinandergeschichtet werden und eine mechanisch feste Verbindung zwischen dem Füllstoff (82) und den Platten (64 bis 67) hergestellt wird, daß anschließend mehrere plattenförmige Formkörper (84) durch in Richtung der Länge L des Stapels (62) aufeinanderfolgende Schnitte senkrecht zur Stapelebene und parallel zur Stapelbreite B hergestellt werden, daß diese Formkörper (84) mit ihren Flachseiten jeweils abwechselnd mit einer dünnen Zwischenschicht aus einem Füllstoff (86), dessen akustische Impedanz wesentlich verschieden ist von der akustischen Impedanz des Ultraschallschwingermaterials der Platten (64 bis 67), aufeinandergeschichtet werden und eine unlösbare Verbindung zwischen dem Füllstoff (86) und den Formkörper (84) hergestellt wird, und daß schließlich jeweils eine die Wandlerelemente (14) enthaltene Matrix (2) durch einen Schnitt senkrecht zur Formkörper-Stapelebene und parallel zur Formkörper-Stapelbreite (H) hergestellt wird.

2. Verfahren nach Anspruch 1, dadurch gekennzeichnet, daß vor dem vollständigen Aushärten des Füllstoffes die Matrix 2 gekrümmt und danach der Füllstoff ausgehärtet wird.

## Claims

1. A process for the manufacture of an ultrasonic transducer arrangement having a matrix of ultrasonic oscillators (4 to 12) which respectively consist of a sub-matrix of column-like transducer elements (14) commonly electrically controlled whose height is substantially greater than their thickness, and in which both the interspaces between the ultrasonic oscillators (4 to 12) and also the interspaces between the transducer elements (14) are filled with a filler (16) whose acoustic impedance is essentially different from the acoustic impedance of the oscillator material of the transducer elements (14) and constitutes a form-locking and mechanically rigid connection between the transducer elements (14), characterised in that plates (64 to 67) made of an ultrasonic oscillator material having flat sides are stacked with their flat sides one upon another in alternation with a thin layer of a filler (82), whose acoustic impedance is essentially different from the acoustic impedance of the ultrasonic oscillator material of the plates (64 to 67), and a mechanically rigid connection is established between the filler (82) and the plates (64 to 67), that a plurality of plate-shaped moulded bodies (84) are subsequently produced through consecutive cuts in the direction of the length L of the stack (62) at right angles to the plane of the stack and parallel to the width B off the stack, that these moulded bodies (84) are stacked one upon another with their flat sides in alternation with a thin intermediate layer of a filler (86) whose acoustic impedance is essentially different from the acoustic impedance of the ultrasonic oscillator material of the plates (64 to 67), and an undetachable connection is established between the filler (86) and the moulded bodies (84), and that a matrix (2) containing the transducer elements (14) is finally produced by a cut at right angles to the plane of the stack relating to the moulded bodies and parallel to the width (H) of the stack relating to the moulded bodies.

2. A process as claimed in Claim 1, characterised in that the matrix 2 is bent prior to the complete hardening of the filler and the filler subsequently fully hardened.

## Revendications

1. Procédé pour fabriquer un ensemble transducteur à ultrasons comportant une matrice d'oscillateurs à ultrasons (4 à 12), qui sont constitués respectivement par une matrice secondaire d'éléments transducteurs (14) en forme de colonnes commandés électriquement en commun et dont la hauteur est nettement supérieure à leur épaisseur, et dans lequel aussi bien les espaces intercalaires compris entre les oscillateurs à ultrasons (4 à 12) que les espaces intercalaires compris entre les éléments transducteurs (14) sont remplis par une substance de remplissage (16), dont l'impédance acoustique diffère nettement de l'impédance acoustique du matériau constitutif des oscillateurs des éléments transducteurs (14) et qui forme une liaison mécaniquement rigide par formes complémentaires entre les éléments transducteurs (14), caractérisé par le fait qu'on empile les unes sur les autres, par leurs faces planes, des plaques (64 à 67) formées en un matériau constitutif des oscillateurs à ultrasons, respectivement en alternance avec des couches minces formées d'une substance de remplissage (82), dont l'impédance acoustique diffère nettement de l'impédance acoustique du matériau constitutif des oscillateurs à ultrasons des plaques (64 à 67), et qu'on établit une liaison mécaniquement rigide entre la substance de remplissage (82) et les plaques (64 à 67), qu'on réalise ensuite plusieurs pièces de formes (84) en plaques grâce à des découpes se succédant suivant la direction de la longueur L de la pile (62), perpendiculairement au plan de la pile et parallèlement à la largeur B de la pile, qu'on empile les unes sur les autres, par leurs faces planes, ces pièces de formes (84) respectivement en alternance avec des couches intercalaires minces constituées par une substance de remplissage (86), dont l'impédance acoustique est nettement différente de l'impédance acoustique du matériau constitutif des oscillateurs à ultrasons des plaques (64 à 67), et qu'on réalise une liaison indétachable entre la substance de remplissage (86) et la pièce de forme (84), et qu'on réalise enfin des matrices respectives (2) contenant les éléments transducteurs (14), au moyen de découpes effectuées perpendiculairement au plan de la pile des pièces de forme et parallèlement à la largeur (H) de la pile de pièces de forme.

2. Procédé suivant la revendication 1, caractérisé par le fait qu'on cintre la matrice (2) avant le durcissement complet de la substance de remplissage et qu'on effectue ensuite le durcissement de cette substance.

FIG 1

FIG 2

FIG 3

FIG 4

FIG 5